# EUROPEAN PATENT SPECIFICATION

(11) **EP 4 349 385 B1**
(45) Date of publication and mention of the grant of the patent: **19.08.2026**
(21) Application number: 22306466.8
(22) Date of filing: 03.10.2022
(51) Int. Cl.: A61M 5/32

(54) **AUTOINJECTOR FOR AUTOMATIC INJECTION OF A PRODUCT INTO AN INJECTION SITE**
AUTOINJEKTOR ZUR AUTOMATISCHEN INJEKTION EINES PRODUKTS IN EINE INJEKTIONSSTELLE
AUTO-INJECTEUR POUR L'INJECTION AUTOMATIQUE D'UN PRODUIT DANS UN SITE D'INJECTION

(43) Date of publication of application: 10.04.2024
(73) Proprietor: Becton Dickinson France, 38800 Le Pont-de-Claix (FR)
(72) Inventor: FIARD, Michael, 38320 Eybens (FR)
(74) Representative: Germain Maureau

(56) References cited:
- WO-A1-2022/078986
- US-A1- 2018 272 075
- US-A1- 2022 111 153

## Description

The present invention relates to an autoinjector.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Also, by outer it is meant centrifugally oriented with regard to a central longitudinal axis, and by inner it is meant centripetally with regard to said central longitudinal axis.

Automatic injection devices are designed for automatic injection of a medical product into an injection site. Autoinjectors usually comprise a housing for receiving a medical container having a barrel defining a reservoir for containing the medical product, the barrel having a distal end provided with an injection needle and an opened proximal end receiving a plunger rod for pushing a stopper. The opened proximal end is usually provided with a flange.

Autoinjectors also include a needle cover moving from an extended to a retracted position to shield or unveil the needle and an injection mechanism for automatically injecting the medical into an injection site. The injection mechanism is usually triggered, directly or indirectly, by the needle cover when the needle cover moves to the retracted position. It is important to prevent untimely triggering or pre-activation of the autoinjector, for example during transport or storage. The needle covers are usually held or urged back in their extended position by a safety spring.

However, to assess the robustness of the autoinjectors, autoinjectors are subjected to drop tests as required in ISO11608. These drop tests usually consist in dropping the autoinjectors at least once from a height of 1 m onto a horizontal floor. There are three drop directions : a drop 'cap upward' (the proximal end of the autoinjector hits the floor first), a drop 'cap downward' (the distal end of the autoinjector hits the floor first), and a drop with the autoinjector being horizontal.

During a drop 'cap upward', the kinetic energy released when the autoinjector hits the ground may cause the needle cover to suddenly move towards the retracted position against the action of the safety spring. There is thus a risk that the autoinjector is inadvertently activated.

There is accordingly a need for an autoinjector that is able to avoid activation of the injection mechanism during a drop test.

Document WO2022078986 discloses a medicament delivery member guard lock assembly. Document US2018272075 discloses a safety assembly for a medicament delivery device.

An aspect of the invention is an autoinjector, for automatic injection of a product into an injection site, said autoinjector including :
a housing extending along longitudinal axis A and having an opened distal end, said housing being configured to receive a medical container having a barrel defining a reservoir for containing a medical product, said barrel having a distal end provided with an injection needle,
a needle cover coupled to and axially movable with respect to said housing between a first extended position, in which the needle cover at least partially shields the injection needle, a retracted position, in which the needle cover moves proximally with respect to the housing to trigger injection of the medical product into an injection site, and a second extended position in which the needle cover moves back in the distal direction to shield the injection needle,
a cap removably attached to the opened distal end of the housing,
a retainer coupled to the housing for blocking a proximal movement of the needle cover from the first extended position to the retracted position as long as the cap is attached to the housing, wherein the retainer includes
a distal abutment surface which is radially movable between a blocking position in which said distal abutment surface abuts against a proximal abutment surface of the needle cover, and a release position in which said distal abutment surface is radially shifted away from the proximal abutment surface of the needle cover to allow movement of the needle cover in the proximal direction, and wherein the cap includes
a locking tab radially abutting against the retainer for maintaining the distal abutment surface of the retainer in the blocking position.

As a result, the autoinjector of the invention is prevented from being activated or pre-activated. As long as the cap is attached to the housing, the retainer cannot move to the release position. Thus, the needle cover cannot move to the retracted position. There is thus no risk that the injection mechanism is triggered. The autoinjector of the invention can thus succesfully pass drop tests. Inadvertent activation of the injection mechanism is avoided.

The proximal abutment surface may be arranged on an outer lateral wall of the needle cover. The locking tab is configured for radially abutting against the retainer so as to lock the retainer in the blocking position.

The autoinjector of the invention may further include some or all of the features below.

In an embodiment, the locking tab has an outer abutment surface abutting against an inner lateral wall of the housing. This helps maintain the locking tab in abutment against the retainer such that the retainer reliably blocks the needle cover.

According to the invention, the retainer defines an accommodation room for receiving a free proximal end of the locking tab between an outer lateral surface of the retainer and an inner lateral wall of the housing.

Preferably, the locking tab has a proximally tapering width.

According to the invention, an outer lateral surface of the free proximal end of the locking tab is separated from the inner lateral wall of the housing by a radial gap. The radial dimension of this gap may be greater than the radial dimension (thickness) of the proximal end of the locking tab.

In an embodiment, the locking tab has an axial rib distally extending from the free proximal end of the locking tab and said axial rib is fixed to an external wall of the retainer.

In an embodiment, the locking tab has an axial rib distally extending from the free proximal end of the locking tab and said axial rib has a distal end which is fixed to a connecting member that connects a cap external wall to a remover of the cap.

In an embodiment, the locking tab has a stiffener arranged at an outer side of the free proximal end, the stiffener proximally protruding from a distal edge of the cap.

These features provide support to the locking tab and distribute the efforts received by the locking tab, thus avoiding deformation of the locking tab. As a result, the retainer is reliably maintained by the locking tab in the blocking position.

In an embodiment, the stiffener of the locking tab includes an inclined surface for easing insertion of the locking tab inside the housing.

In an embodiment, the retainer includes a flexible leg, said flexible leg having a fixed end secured to the housing and a free end defining the distal abutment surface of the retainer. The flexible leg permits movement of the retainer to the release position under the force exerted by the user or by the safety spring on the needle cover after removal of the cap.

In an embodiment, the free end of the flexible leg is distal to the fixed end. Therefore, the retainer acts as a ratchet. This provides a better resistance to any proximal movement of the needle cover during a drop test.

Possibly, the opened distal end of the housing is distal to the free end of the flexible leg.

Possibly, the free distal end is closer to the central longitudinal axis than the fixed end of the retainer, at least when the retainer is in the blocking position.

In an embodiment, the fixed end of the flexible leg includes an annular rib radially protruding from an inner lateral wall of the housing. This eases movement of the retainer from the blocking position to the release position after removal of the cap. This also helps define the accommodation room between the retainer and the housing for receiving the locking tab.

In an embodiment, the blocking position of the retainer is a rest position of the flexible leg. That is, the flexible leg is in its natural (not deformed) state in the blocking position. This makes the blocking action of the retainer more reliable.

In an alternative embodiment, the release position of the retainer is a rest position. That is, the flexible leg is in its natural (not deformed) state in the release position. The retainer thus has to be pressed against the needle cover to stay in the blocking position. However, this limits the risks that the retainer inadvertently blocks the needle cover after the injection process when the needle cover moves back in the distal direction towards the second extended position (safety position).

In both embodiments, movement of the distal abutment of the retainer from the blocking to the release position may be a radial outward movement.

In an embodiment, the retainer has a proximal abutment surface abutting against a distal abutment surface of the needle cover for blocking the needle cover in the distal direction. Thus, the needle cover is locked in the first extended position as long as the cap is attached to the housing. The distal and/or the proximal abutment surface of the retainer may be inclined with regard to the central longitudinal axis A so that the retainer moves to the release position under the pressure of the end user or the action of the safety spring. The distal and/or proximal abutment surface of the needle cover may also be inclined with regard to the longitudinal axis A.

In an embodiment, the autoinjector includes securing means for removably securing the cap to the housing in both axial and rotational directions. This avoids inadvertent disengagement of the locking tab and the retainer.

Preferably, the securing means include an orthoradial shoulder of the housing abutting against an opposite orthoradial edge of the cap. This prevents rotation of the cap relative to the housing when the cap is attached to the housing, so that the locking tab cannot rotationally disengage the retainer.

The orthoradial shoulder of the housing abutting against an opposite orthoradial edge of the cap also form keyed elements for allowing proper positioning of the cap relative to the housing. Thus, the locking tab of the cap reliably engages the retainer of the housing when the cap is being mounted onto the housing.

The invention and the advantages arising therefrom will clearly emerge from the detailed description that is given below with reference to the appended drawings as follows :
- Figure 1 is a perspective view of an autoinjector according to an embodiment of the invention,
- Figures 2A and 2B are perspective views of a cap of an autoinjector according to an embodiment of the invention,
- Figure 3 is a bottom view of an autoinjector according to an embodiment of the invention,
- Figure 4A is a cross-section view of an autoinjector according to an embodiment of the invention, in the A-A plane of the Figure 3,
- Figure 4B is a cross-section view of an autoinjector according to an embodiment of the invention, in the B-B plane of the Figure 3,
- Figure 5A is a cross-section view of an autoinjector according to an embodiment of the invention, in the A-A plane of the Figure 3, when the cap is removed from the housing,
- Figure 5B is a cross-section view of an autoinjector according to an embodiment of the invention, in the B-B plane of the Figure 3, when the cap is removed from the housing,
- Figure 6A is a cross-section view of an autoinjector according to an embodiment of the invention, without the cap,
- Figure 6B is a cross-section of the autoinjector of Figure 6A, the cap being attached to the housing,
- Figures 7A, 7B, 7C are cross-section views of an autoinjector according to an embodiment of the invention, the needle cover being, respectively, in the first extended position (ready-to-use position), the retracted position, and the second extended position (safety position),
- Figure 8 is a perspective cross-section view of a retainer of an autoinjector according to an embodiment of the invention,
- Figure 9A is a perspective view of an autoinjector according to an embodiment of the invention, the cap being removed from the housing,
- Figure 9B is a partial cross-section and perspective view of a cap of an autoinjector according to an embodiment of the invention.

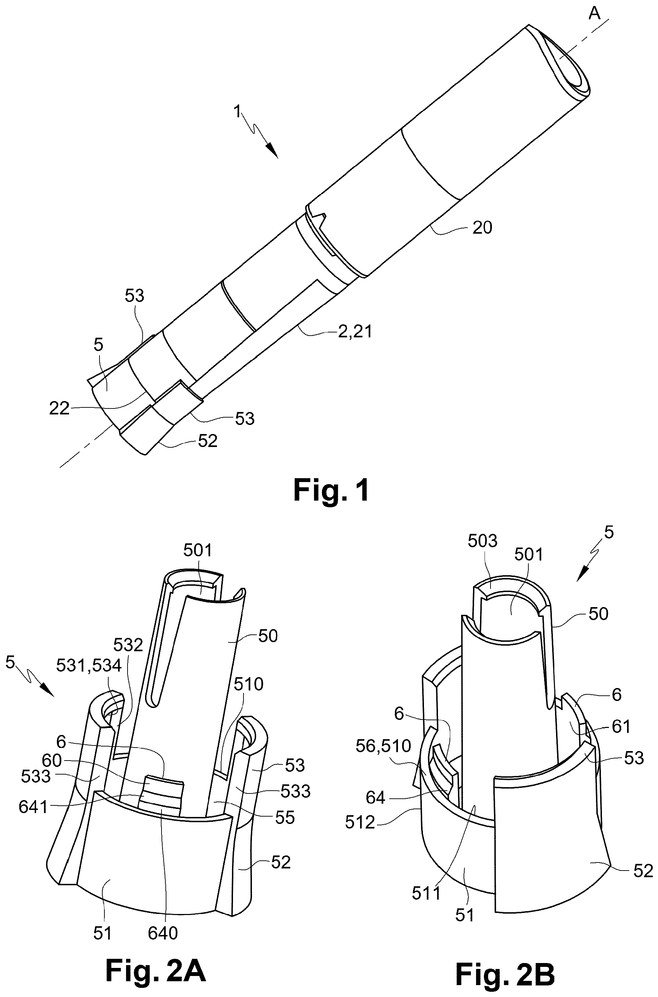

With reference to Figure 1 is shown an autoinjector 1 according to an embodiment of the invention. The autoinjector 1 is designed for automatic injection of a product into an injection site. The autoinjector 1 extends along a longitudinal axis A. The autoinjector 1 includes a housing 2 comprising a top body 20 and a bottom body 21 assembled to each other by any appropriate securing means such as, for instance, snap-fitting means, a cap 5 removably attached to the housing 2, and a needle cover 4 axially movable within the housing 2.

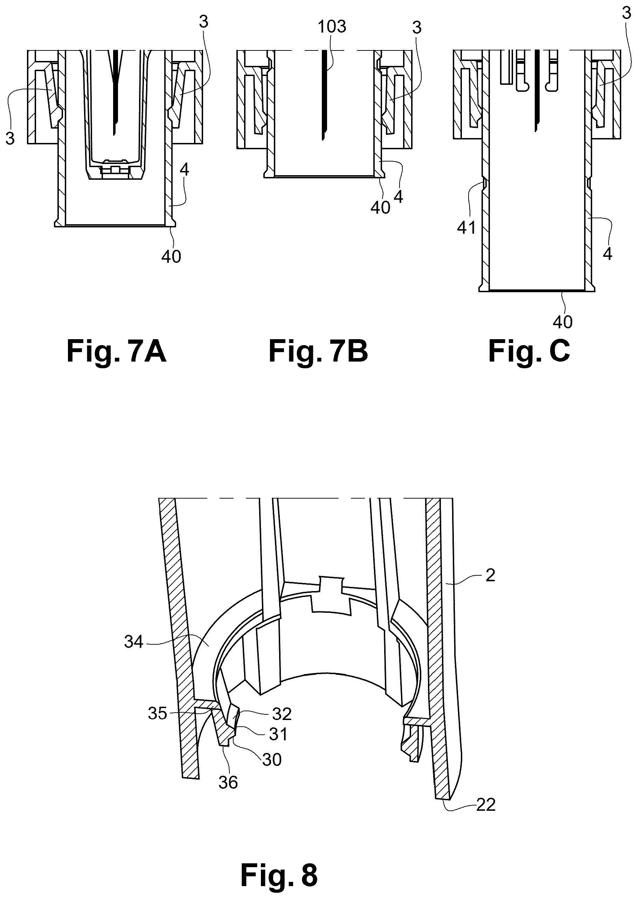

The needle cover 4 is arranged within the bottom body 21 and a safety spring 45 (not shown) for urging the needle cover 4 in a distal direction. With reference to Figures 7A, 7B, 7C, the needle cover 4 is axially movable along the longitudinal axis A with respect to the bottom body 21 between a first extended position (ready-to-use position, Figure 7A), in which the needle cover 4 at least partially or completely shields the injection needle 103, a retracted position (activated position, Figure 7B), proximally located relative to said first extended position, in which the needle cover 4 moves inside the autoinjector 1 to activate the injection mechanism and thus trigger the injection operation (in which the injection spring moves the plunger rod in the distal direction, thus pushing the stopper 105 to expel the medical product contained within the barrel 101), and a second extended position (safety position, Figure 7C) in which after injection the needle cover 4 moves back in the distal direction to safely shield the injection needle 103.

The needle cover 4 has a distal end designed to be pressed against an injection site. Movement of the needle cover 4 from the first extended position (Figure 7A) to the retracted position (Figure 7B) is thus caused by the user pressing the distal end 40 of the needle cover 4 against the injection site, and movement of the needle cover 4 from the retracted position (Figure 7B) to the safety position (Figure 7C) is due to the safety spring 45 pushing the needle cover 4 back in the distal direction.

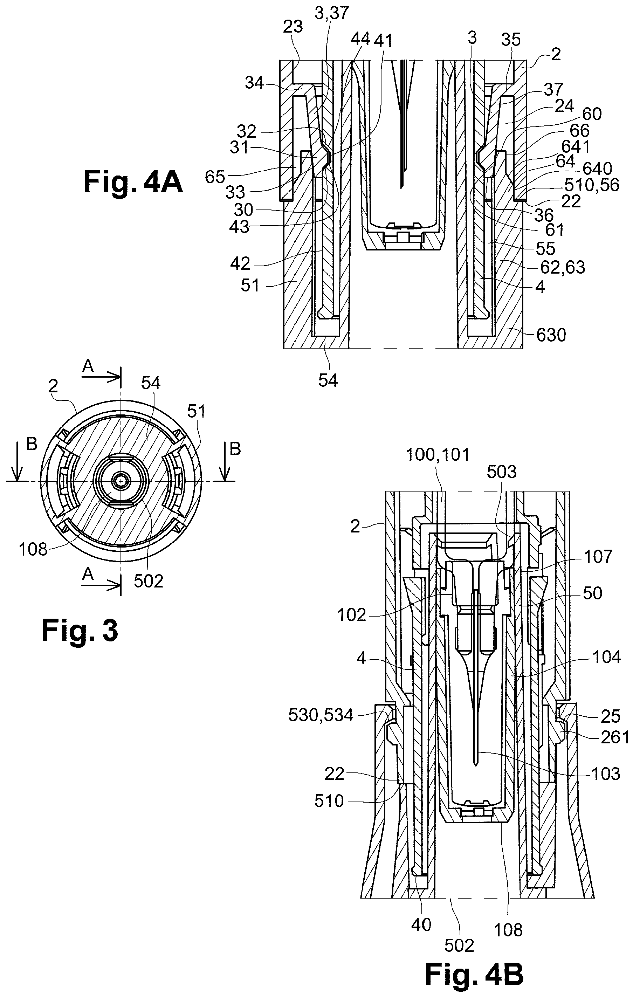

With reference to Figure 4A, the needle cover 4 further includes a retaining feature, such as a radial recess 41, arranged on an outer lateral surface 42 of the needle cover 4 for retaining the needle cover 4 in the first extended position, as will be explained in further details below. The radial recess 41 defines a proximal abutment surface 43, and may further define an opposite distal abutment surface 44.

The housing 2 (more specifically the bottom body 21) is configured to receive a medical container 100 such as a syringe, for instance a prefilled syringe. The medical container 100 has a tubular barrel 101 defining a reservoir for containing a medical product to be injected. The barrel 101 has a distal end 102 provided with an injection needle 103 and a neddle shield removably attached to said distal end for protecting and sealing the injection needle 103. The barrel 101 has an opened proximal end (not shown) which may be provided with a flange and which is configured to receive a plunger rod urged in the distal direction by an injection spring. The plunger rod is configured to push a stopper 105 arranged inside the barrel 101, thereby expelling the medical product. The top body 20 of the housing 2 receives an injection mechanism which includes the plunger rod, the injection spring and any other appropriate means known from the art for triggering and performing an injection operation.

The housing 2 (more specifically the bottom body 21) has an opened distal end, as shown for instance in Figure 4A, and a retainer 3 for retaining the needle cover 4 in the first extended position. The retainer 3 extends from an inner lateral wall 23 of the housing 2 for engaging the retaining feature, i.e. the radial recess 41, of the needle cover 4. The retainer 3 is preferably arranged at the distal end 22 of the housing 2 to cooperate with a locking tab 6 of the cap 5, as will be explained below.

The retainer 3 includes a distal abutment surface configured for abutting against the proximal abutment surface 43 of the needle cover 4. The abutment of the distal abutment surface 30 of the retainer 3 against the proximal abutment surface 43 of the needle cover 4 prevents the needle cover 4 from moving to the retracted position. That is, the distal abutment surface 30 of the retainer 3 blocks a proximal movement of the needle cover 4. The distal abutment surface 30 of the retainer 3 and the proximal abutment surface 43 of the needle cover 4 may be inclined with regard to the central longitudinal axis A. Therefore, the retainer 3 allows for movement of the needle cover 4 in the proximal direction when the proximal force exerted on a distal end of said needle cover 4 exceeds a predetermined threshold, i.e. when the distal end 40 of the needle cover 4 is pressed by a user against the injection site to perform the injection operation, but blocks a proximal movement should the needle cover 4 be accidentally urged in the proximal direction, i.e. for instance if the autoinjector 1 falls down on the floor cap 5 upward. The distal abutment surface may be arranged at a distal side of an inward radial protrusion 31. The protrusion of the retainer 3 and the radial recess 41 of the needle cover 4 may be complementarily shaped. The proximal abutment surface 32 of the retainer 3 may be formed at a proximal side of the protrusion.

The retainer 3 may further include a proximal abutment surface, which may be arranged at a proximal side of the protrusion, for abutting against the distal abutment surface 44 of the needle cover 4 in order to lock the needle cover 4 in the first extended position.

The retainer 3 has an outer lateral surface 33 arranged opposite said protrusion for receiving a locking tab 6 of the cap 5. The locking tab 6 thus exerts a radial inward force against said outer lateral surface 33 for keeping the retainer 3 of the housing 2 engaged with the needle cover 4.

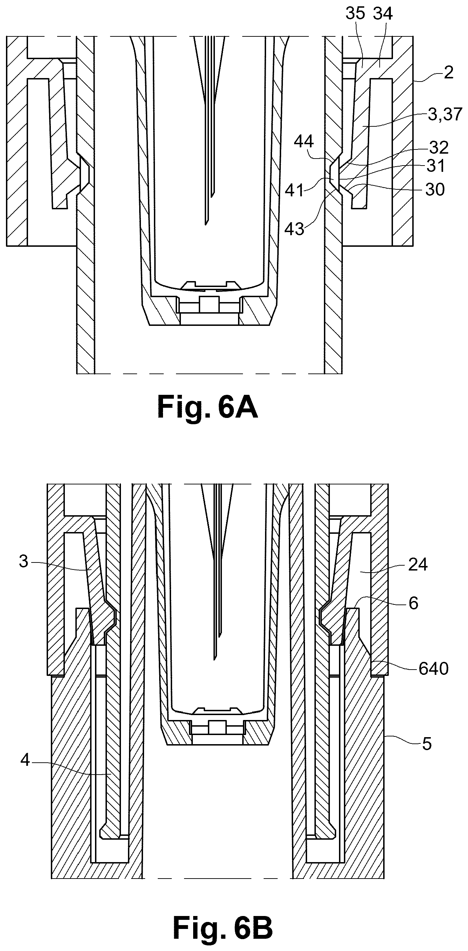

The retainer 3 is outwardly radially movable with respect to the needle cover 4, between an axial blocking position (Figure 4A), in which the distal abutment surface 30 of the retainer 3 axially abuts against the proximal abutment surface 43 of the needle cover 4, and a release position (Figure 6A), in which the distal abutment surface 30 of the retainer 3 is shifted away from the axial path of the proximal abutment of the needle cover 4 such that the needle cover 4 moves towards the retracted position due to the pressure exerted by its distal end on the injection site.

The retainer 3 may be in the form of a flexible leg 37 radially deformable between the blocking position and the release position. In the embodiment illustrated in Figures 4A-4B, the blocking position is a rest (non deformed) position of the retainer 3, while the release position is a deformed position of the retainer 3.

The retainer 3 may include a circumferential rib 34 for connecting the (fixed end 35 of the) flexible leg 37 to the inner lateral wall 23 of the housing 2. The circumferential rib 34 also helps delimit an accommodation room 24 for receiving the locking tab 6 of the cap 5 without risk of blockage of the locking tab 6.

The flexible leg 37 of the retainer 3 has a fixed end 35 secured to the housing 2, and an opposite free end which may include the protrusion. The free end includes, on an inner side, the distal abutment of the retainer 3, and on an outer side, the outer lateral surface 33 that receives the locking tab 6. Preferably, the free end is distally located with respect to the fixed end 35. Thus, the retainer 3 acts as a ratchet that prevents movement of the needle cover 4 in the proximal direction. The retainer 3 may be inclined such that, in the blocking position, the free end is closer to the central longitudinal axis A than the fixed end 35. As a result, the accommodation room 24 defined between the retainer 3 and the housing 2 widens in the distal direction. This allows reliable insertion of the locking tab 6 in the accommodation when the cap 5 is attached to the housing 2.

Preferably, the free end 36 of the retainer 3 is proximally located with respect to the distal end 22 of the housing 2. That is, the retainer 3 is fully contained within the housing 2 and does not distally extend outside the housing 2, beyond the distal end of said housing 2.

The retainer 3 is secured to the housing 2 by means of the fixed end 35 and, if need be, of the circumferential rib 34. That is, the retainer 3 is integral with the housing 2. Preferably, the retainer 3 and the housing 2 are made of a single piece.

The cap 5 of the autoinjector 1 is removably attached to the housing 2 in order to close the opened distal end 22 of the bottom body 21. The cap 5 includes a remover 50 for removing the needle shield 104, an external wall 51 extending around the remover 50 for protecting the remover 50 and for securing the cap 5 to the housing 2, and a connecting member 54 for rigidly connecting the remover 50 to the external wall 51.

The remover 50 is arranged within the external wall 51 for engaging the needle shield 104 when the cap 5 is attached to the housing 2, and is configured for removing the needle shield 104 when the cap 5 is removed from the housing 2. The remover 50 may be in the form of an axial sleeve having an opened proximal end 501 for receiving the needle shield 104, and an opposite opened distal end 502, Figure 3, giving access to a distal end 108 of the needle shield 104 for performing a push-back step of the medical container 100 during assembly of the autoinjector 1. The remover 50 includes a hook 503 or any appropriate means for engaging a proximal end 107 of the needle shield 104 such that removal of the cap 5 from the housing 2 by the user entails removal of the needle shield 104 from the medical container 100, thereby uncovering the injection needle 103 before injection.

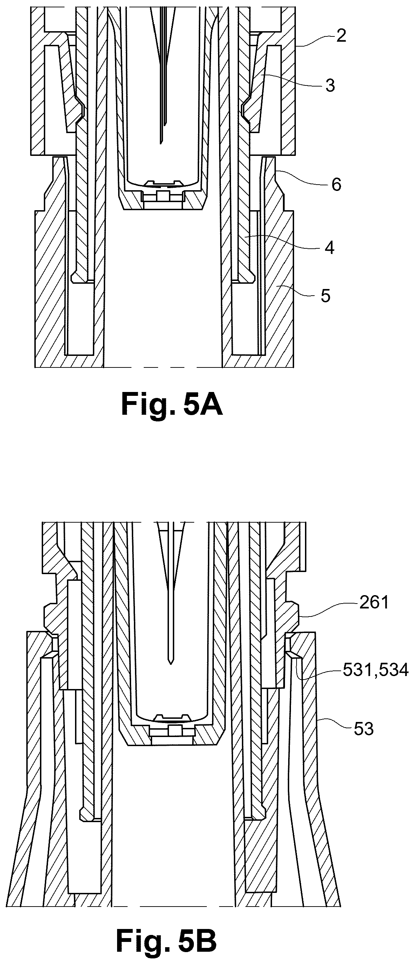

The remover 50 is connected to the external wall 51 by means of the connecting member 54, which may arranged at the distal end of the cap 5. The connecting member 54 may be a disk or end-plate which is in the form of a transversal wall orthogonal to the longitudinal axis A. The connecting member 54 more specifically connects the distal end of the remover 50 to the distal end of the external wall 51. The connecting member 54 provides a distance between the remover 50 and the external wall 51, such that the remover 50 and the external wall 51 delimit a circumferential cavity 55 for accommodating the needle cover 4, Figure 5A.

The external wall 51 may include grasping means, for instance in the form of two diametrically opposite outer grasping surfaces 52 which are inclined relative to the longitudinal axis A for easing withdrawal of the cap 5. As illustrated in Figures 2A-2B, the grasping surfaces 52 may taper in a proximal direction.

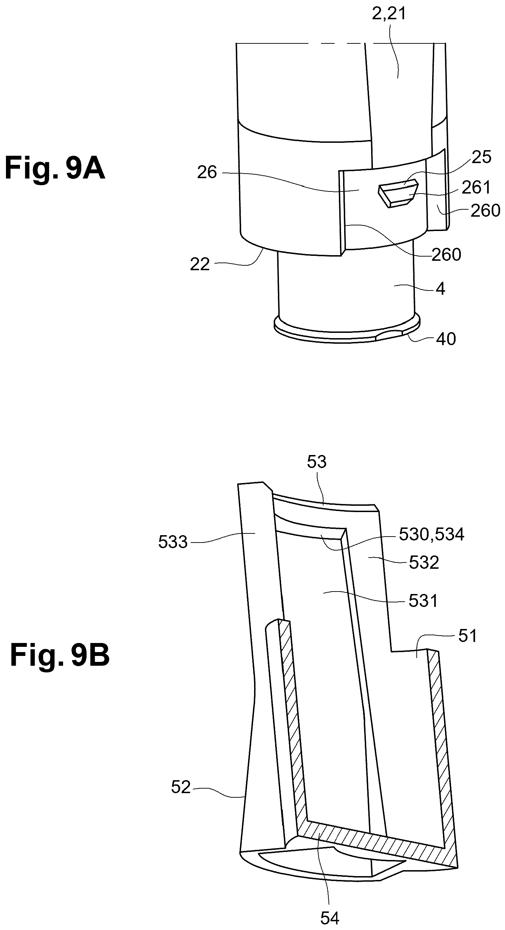

The external wall 51 further includes securing means for removably securing the cap 5 to the housing 2. The securing means may be configured for securing the cap 5 in both the axial and the rotational directions. In the embodiment illustrated in Figures 2A-2B, the securing means include two securing legs 53 proximally protruding from a distal edge 510 of the external wall 51. The distal edge 510 of the external wall 51 is configured for abutting against the distal end 22 of the housing 2 to stop the cap 5 in the proximal direction. The two diametrically opposite securing legs 53 preferably extend in the continuity of the grasping surfaces 52, i.e. they run alongside the grasping surfaces 52. The securing legs 53 include a distally oriented stop 530, which may be arranged at a proximal end of the securing legs 53 for abutting against a proximally oriented stop 25 of the housing 2, Figure 9A. The distally oriented stop 530 may be a distal shoulder 534 defined by a radial recess 531 extended through an inner lateral surface 532 of the securing legs 53, Figure 9B. The housing 2 may include an external recess 26 for receiving the securing legs 53. The external recess 26 of the housing 2 and the securing legs 53 may be complementarily shaped. The proximally oriented stop 25 of the housing 2 may be arranged within the external recess 26 for engaging the complementary shaped distally oriented stop 530 of the securing legs 53. The proximal stop may be in the form of a radial protrusion 261 which may taper in the distal direction to favor attachment of the cap 5 to the housing 2.

Still with reference to Figures 2, 9A and 9B, the securing legs 53 define an orthoradial edge 533 for abutting against a corresponding orthoradial shoulder 260 of the external recess 26 of the housing 2. This prevents any rotation of the cap 5 when the cap 5 is attached to the housing 2. The orthoradial edge 533s and shoulders also provide indication of the appropriate rotational position of the cap 5 relative to the housing 2 that allows for engagement of a locking tab 6 of the cap 5 with the retainer 3 of the housing 2 when the cap 5 is being mounted to the housing 2.

The external wall 51 of the cap 5 includes a locking tab 6 which proximally extends above the distal edge 510 of the external wall 51 for locking the retainer 3 of the housing 2 in the blocking position. The locking tab 6 may be in the form of two diametrically opposite locking tabs 6 which may be arranged at 90° from the securing legs 53. The locking tabs 6 include a supporting portion 62 for securing the locking tabs 6 to the cap 5, and a locking portion for locking the retainer 3 in the axial blocking position.

The supporting portion 62 may include an axial rib 63 secured to an inner lateral surface 511 of the external wall 51 and radially inwardly protruding from said inner lateral surface 511, and/or a stiffener 64 secured to the distal edge 510 of the external wall 51, and/or a distal end 630 secured to a proximal side of the connecting member 54. The stiffener 64 includes an outer abutment surface 640 for abutting against the inner lateral wall 23 of the housing 2. The stiffener 64 may also include a chamfer 641 or any other inclined surface that may abut against the distal end 22 of the housing 2 for easing insertion of the locking tabs 6 into the accommodation room 24s when the cap 5 is being mounted onto the housing 2. The stiffener 64 proximally protrudes from the distal edge 510 of the external wall 51, but may however remains distant from an outer lateral surface 512 of the external wall 51 so that a proximal shoulder 56 is defined between the stiffener 64 and the outer lateral surface 512 to abut against the distal end 22 of the housing 2.

The locking portion may be arranged at a free proximal end 60 of the locking tabs 6 for engaging inside the accommodation room 24 and abutting against the outer lateral surface 33 of the retainer 3, thereby locking the retainer 3 in the axial blocking position. To that end, the locking portion includes an inner side surface 61 that abuts against the outer lateral surface 33 of the retainer 3. The locking portion may be distant from the inner lateral wall 23 of the housing 2 and may thus delimit a radial gap 65 between the housing 2 and the locking tab 6.

It is contemplated that the remover 50, the external wall 51, the connecting member 54 and the locking tabs 6 of the cap 5 may be integral with each other. In the embodiment illsutrated for instance in Figures 2A, 2B or 4A, the remover 50, the external wall 51, the connecting member 54 and the locking tabs 6 may be made of a single piece.

The operation of the autoinjector 1 is described below.

In Figure 4A, the cap 5 is attached to the housing 2. The retainer 3 is in the blocking position: the retainer 3 engages the needle cover 4 and axially blocks the needle cover 4 in the pre-use position. If the autoinjector 1 falls down on the floor, the needle cover 4 is thus prevented from moving to the retracted position and the autoinjector 1 is thus prevented from being inadvertently activated. The locking tabs 6 of the cap 5 abut againts the retainer 3, thereby keeping the retainer 3 in the blocking position as long as the cap 5 is attached to the housing 2.

To perform an injection operation, the user has to grasp the cap 5 and pull in the distal direction to remove the cap 5 from the housing 2, Figure 9A. Since the remover 50 of the cap 5 is engaged with the needle shield 104, removal of the cap 5 entails removal of the needle shield 104. Besides, the locking tabs 6 are removed together with the cap 5, so that the retainer 3 is able to move to the release position. Since the blocking position here is a rest position of the retainer 3, then the retainer 3 remains engaged with the needle cover 4 (Figures 5A, 7A).

The user presses the distal end 40 of the needle cover 4 against the injection site. The force exerted by the user causes the needle cover 4 to outwardly radially deflect the retainer 3. The retainer 3 moves to the release position and disengages the needle cover 4. The needle cover 4 moves to the retracted position (Figure 7B) and triggers the injection mechanism.

When the injection is completed, the user withdraws the autoinjector 1 from the injection site. Due to the safety spring 45, the needle cover 4 moves back in the distal direction and passes over the retainer 3 until the needle cover 4 reaches the safety position (Figure 7C).

Figures 6A-6B illustrate another embodiment wherein the blocking position of the retainer 3 corresponds to a deformed position of the retainer 3, while the release position corresponds to a rest (not deformed) position of the retainer 3. That is, the retainer 3 naturally remains in the release position as visible in Figure 6A. In order to lock the needle cover 4, the retainer 3 must be deformed to the blocking position by means of the locking tabs 6 of the cap 5 which exert a radial inward force on the retainer 3 (Figure 6B). As long as the cap 5 is attached to the housing 2, the locking tabs 6 maintain the retainer 3 engaged with the needle cover 4 in the axial blocking position.

The operation of the autoinjector 1 according to the embodiment of Figures 6A-6B is similar to the embodiment of Figures 4A-4B, except that the blocking position of the retainer 3 being the deformed position of said retainer 3, the flexible legs 37 of the retainer 3 automatically flex back to their rest position when the cap 5 is removed from the housing 2. Then, the retainer 3 does not interfere any longer with the needle cover 4.

It is readily understandable from the above description that the autoinjector 1 of the invention permits to prevent inadvertent activation of the injection mechanism during a drop test as long as the cap 5 is attached to the housing 2.

It is to be understood that the present invention is not limited to the embodiments described above and illustrated in the drawings; rather, the skilled person will recognize that many changes and modifications may be made within the scope of the appended claims.

## Claims

1. An autoinjector (1), for automatic injection of a product into an injection site, said autoinjector (1) including :
a housing (2) extending along longitudinal axis A and having an opened distal end (22), said housing (2) being configured to receive a medical container (100) having a barrel (101) defining a reservoir for containing a medical product, said barrel (101) having a distal end (102) provided with an injection needle (103),
a needle cover (4) coupled to and axially movable with respect to said housing (2) between a first extended position, in which the needle cover (4) at least partially shields the injection needle (103), a retracted position, in which the needle cover (4) moves proximally with respect to the housing (2) to trigger injection of the medical product into an injection site, and a second extended position in which the needle cover (4) moves back in the distal direction to shield the injection needle (103),
a cap (5) removably attached to the opened distal end (22) of the housing (2),
a retainer (3) coupled to the housing (2) for blocking a proximal movement of the needle cover (4) from the first extended position to the retracted position as long as the cap (5) is attached to the housing (2), wherein the retainer (3) includes
a distal abutment surface (30) which is radially movable between a blocking position in which said distal abutment surface (30) abuts against a proximal abutment surface (43) of the needle cover (4), and a release position in which said distal abutment surface (30) is radially shifted away from the proximal abutment surface (43) of the needle cover (4) to allow movement of the needle cover (4) in the proximal direction, and wherein the cap (5) includes
a locking tab (6) radially abutting against the retainer (3) for maintaining the distal abutment surface (30) of the retainer (3) in the blocking position,
wherein the retainer (3) defines an accommodation room (24) for receiving a free proximal end (60) of the locking tab (6) between an outer lateral surface (33) of the retainer (3) and an inner lateral wall (23) of the housing (2), and
**characterised in that** an outer lateral surface (66) of the free proximal end (60) of the locking tab (6) is separated from an inner lateral wall (23) of the housing (2) by a radial gap (65).

2. The autoinjector according to the preceding claim, wherein the locking tab (6) has an outer abutment surface (640) abutting against an inner lateral wall (23) of the housing (2).

3. The autoinjector (1) according to any of the preceding claims 1 or 2, wherein the locking tab (6) has an axial rib (63) distally extending from the free proximal end (60) of the locking tab (6) and said axial rib (63) is fixed to an external wall (51) of the retainer (3).

4. The autoinjector (1) according to the preceding claim, wherein the axial rib (63) has a distal end (630) which is fixed to a connecting member (54) that connects a cap (5) external wall (51) to a remover (50) of the cap (5).

5. The autoinjector (1) according to any of the preceding claims 1-4, wherein the locking tab (6) has a stiffener (64) arranged at an outer side of the free proximal end (60), the stiffener (64) proximally protruding from a distal edge (510) of the cap (5).

6. The autoinjector (1) according to the preceding claim, wherein the stiffener (64) includes an inclined surface (641) for easing insertion of the locking tab (6) inside the housing (2).

7. The autoinjector (1) according to any of the preceding claims, wherein the retainer (3) includes a flexible leg (37), said flexible leg (37) having a fixed end (35) secured to the housing (2) and a free end (36) defining the distal abutment surface (30) of the retainer (3).

8. The autoinjector (1) according to the preceding claim, wherein the free end (36) of the flexible leg (37) is distal to the fixed end (35).

9. The autoinjector (1) according to any of the preceding claims 7-8, wherein the fixed end (35) of the flexible leg (37) includes an annular rib (34) radially protruding from an inner lateral wall (23) of the housing (2).

10. The autoinjector (1) according to any of the preceding claims 7-9, wherein the blocking position of the retainer (3) is a rest position of the flexible leg (37).

11. The autoinjector (1) according to any of the preceding claims 7-9, wherein the release position of the retainer (3) is a rest position.

12. The autoinjector (1) according to any of the preceding claims, wherein the retainer (3) has a proximal abutment surface (32) abutting against a distal abutment surface (44) of the needle cover (4) for blocking the needle cover (4) in the distal direction.

13. The autoinjector (1) according to any of the preceding claims, wherein the autoinjector (1) includes securing means for removably securing the cap (5) to the housing (2) in both axial and rotational directions.

## Patentansprüche

1. Autoinjektor (1) zum automatischen Injizieren eines Produkts in eine Injektionsstelle, wobei der Autoinjektor (1) Folgendes enthält:
ein Gehäuse (2), das sich entlang der Längsachse A erstreckt und ein geöffnetes distales Ende (22) aufweist, wobei das Gehäuse (2) so eingerichtet ist, dass es einen medizinischen Behälter (100) aufnimmt, der einen Zylinder (101) aufweist, der ein Reservoir zur Aufnahme eines medizinischen Produkts definiert, wobei der Zylinder (101) ein distales Ende (102) aufweist, das mit einer Injektionsnadel (103) versehen ist,
eine in Bezug auf das Gehäuse (2) gekoppelte und axial bewegliche Nadelabdeckung (4) zwischen einer ersten ausgefahrenen Position, in der die Nadelabdeckung (4) die Injektionsnadel (103) mindestens teilweise abschirmt, einer zurückgezogenen Position, in der sich die Nadelabdeckung (4) proximal in Bezug auf das Gehäuse (2) bewegt, und einer zweiten ausgefahrenen Position, in der sich die Nadelabdeckung (4) in distaler Richtung zurückbewegt, um die Injektionsnadel (103) abzuschirmen,
eine am geöffneten distalen Ende (22) des Gehäuses (2) abnehmbar befestigte Kappe (5),
eine mit dem Gehäuse (2) gekoppelte Halterung (3) zum Blockieren einer proximalen Bewegung der Nadelabdeckung (4) von der ersten ausgefahrenen Position in die zurückgezogene Position, solange die Kappe (5) am Gehäuse (2) befestigt ist, wobei die Halterung (3)
eine distale Anschlagfläche (30) enthält, die radial zwischen einer Blockierposition, in der die distale Anschlagfläche (30) an einer proximalen Anschlagfläche (43) der Nadelabdeckung (4) anliegt, und einer Freigabeposition beweglich ist, in der die distale Anschlagfläche (30) radial von der proximalen Anschlagfläche (43) der Nadelabdeckung (4) weg verschoben ist, um eine Bewegung der Nadelabdeckung (4) in proximaler Richtung zu ermöglichen, und wobei die Kappe (5)
eine Verriegelungslasche (6) enthält, die radial an der Halterung (3) zum Halten der distalen Anschlagfläche (30) der Halterung (3) in der Blockierposition anliegt,
wobei die Halterung (3) einen Aufnahmeraum (24) zur Aufnahme eines freien proximalen Endes (60) der Verriegelungslasche (6) zwischen einer äußeren Seitenfläche (33) der Halterung (3) und einer inneren Seitenwand (23) des Gehäuses (2) definiert, und
**dadurch gekennzeichnet, dass** eine äußere Seitenfläche (66) des freien proximalen Endes (60) der Verriegelungslasche (6) durch einen radialen Spalt (65) von einer inneren Seitenwand (23) des Gehäuses (2) getrennt ist.

2. Autoinjektor nach dem vorhergehenden Anspruch, wobei die Verriegelungslasche (6) eine äußere Anschlagfläche (640) aufweist, die an einer inneren Seitenwand (23) des Gehäuses (2) anliegt.

3. Autoinjektor (1) nach einem der vorhergehenden Ansprüche 1 oder 2, wobei die Verriegelungslasche (6) eine axiale Rippe (63) aufweist, die sich distal vom freien proximalen Ende (60) der Verriegelungslasche (6) erstreckt, und die axiale Rippe (63) an einer Außenwand (51) der Halterung (3) befestigt ist.

4. Autoinjektor (1) nach dem vorhergehenden Anspruch, wobei die axiale Rippe (63) ein distales Ende (630) aufweist, das an einem Verbindungselement (54) befestigt ist, das eine Außenwand (51) der Kappe (5) mit einem Abzieher (50) der Kappe (5) verbindet.

5. Autoinjektor (1) nach einem der vorhergehenden Ansprüche 1 bis 4, wobei die Verriegelungslasche (6) eine Versteifung (64) aufweist, die an einer Außenseite des freien proximalen Endes (60) angeordnet ist, wobei die Versteifung (64) proximal aus einer distalen Kante (510) der Kappe (5) herausragt.

6. Autoinjektor (1) nach dem vorhergehenden Anspruch, wobei die Versteifung (64) eine geneigte Fläche (641) zum Erleichtern des Einführens der Verriegelungslasche (6) in das Gehäuse (2) enthält.

7. Autoinjektor (1) nach einem der vorhergehenden Ansprüche, wobei die Halterung (3) einen flexiblen Schenkel (37) enthält, wobei der flexible Schenkel (37) ein am Gehäuse (2) gesichertes festes Ende (35) und ein freies Ende (36) aufweist, das die distale Anschlagfläche (30) der Halterung (3) definiert.

8. Autoinjektor (1) nach dem vorhergehenden Anspruch, wobei das freie Ende (36) des flexiblen Schenkels (37) distal zum festen Ende (35) ist.

9. Autoinjektor (1) nach einem der vorhergehenden Ansprüche 7 bis 8, wobei das feste Ende (35) des flexiblen Schenkels (37) eine ringförmige Rippe (34) enthält, die radial aus einer inneren Seitenwand (23) des Gehäuses (2) herausragt.

10. Autoinjektor (1) nach einem der vorhergehenden Ansprüche 7 bis 9, wobei die Blockierposition der Halterung (3) eine Ruheposition des flexiblen Schenkels (37) ist.

11. Autoinjektor (1) nach einem der vorhergehenden Ansprüche 7 bis 9, wobei die Freigabeposition der Halterung (3) eine Ruheposition ist.

12. Autoinjektor (1) nach einem der vorhergehenden Ansprüche, wobei die Halterung (3) eine proximale Anschlagfläche (32) aufweist, die an einer distalen Anschlagfläche (44) der Nadelabdeckung (4) zum Blockieren der Nadelabdeckung (4) in distaler Richtung anliegt.

13. Autoinjektor (1) nach einem der vorhergehenden Ansprüche, wobei der Autoinjektor (1) Sicherungsmittel zur abnehmbaren Befestigung der Kappe (5) an dem Gehäuse (2) sowohl in axialer als auch in Rotationsrichtung aufweist.

## Revendications

1. Auto-injecteur (1), pour l'injection automatique d'un produit dans un site d'injection, ledit auto-injecteur (1) comprenant :
un boîtier (2) s'étendant le long d'un axe longitudinal A et ayant une extrémité distale ouverte (22), ledit boîtier (2) étant configuré pour recevoir un récipient médical (100) ayant un cylindre (101) définissant un réservoir pour contenir un produit médical, ledit cylindre (101) ayant une extrémité distale (102) munie d'une aiguille d'injection (103),
un couvercle d'aiguille (4) couplé audit boîtier (2) et mobile de manière axiale par rapport à celui-ci entre une première position étendue, dans laquelle le couvercle d'aiguille (4) protège au moins partiellement l'aiguille d'injection (103), une position rétractée, dans laquelle le couvercle d'aiguille (4) se déplace de manière proximale par rapport au boîtier (2) pour déclencher l'injection du produit médical dans un site d'injection, et une seconde position étendue dans laquelle le couvercle d'aiguille (4) se déplace en arrière dans la direction distale pour protéger l'aiguille d'injection (103),
un capuchon (5) fixé de manière amovible à l'extrémité distale ouverte (22) du boîtier (2),
un dispositif de retenue (3) couplé au boîtier (2) pour bloquer un mouvement proximal du couvercle d'aiguille (4) de la première position étendue à la position rétractée tant que le capuchon (5) est fixé au boîtier (2), où le dispositif de retenue (3) comprend
une surface de butée distale (30) qui est mobile radialement entre une position de blocage dans laquelle ladite surface de butée distale (30) vient en butée contre une surface de butée proximale (43) du couvercle d'aiguille (4), et une position de libération dans laquelle ladite surface de butée distale (30) est écartée radialement de la surface de butée proximale (43) du couvercle d'aiguille (4) pour permettre le mouvement du couvercle d'aiguille (4) dans la direction proximale, et où le capuchon (5) comprend
une languette de verrouillage (6) venant en butée radialement contre le dispositif de retenue (3) afin de maintenir la surface de butée distale (30) du dispositif de retenue (3) dans la position de blocage,
dans lequel le dispositif de retenue (3) définit une chambre de logement (24) pour recevoir une extrémité proximale libre (60) de la languette de verrouillage (6) entre une surface latérale externe (33) du dispositif de retenue (3) et une paroi latérale interne (23) du boîtier (2), et
**caractérisé en ce que**
une surface latérale externe (66) de l'extrémité proximale libre (60) de la languette de verrouillage (6) est séparée d'une paroi latérale interne (23) du boîtier (2) par un espace radial (65).

2. Auto-injecteur selon la revendication précédente, dans lequel la languette de verrouillage (6) a une surface de butée externe (640) venant en butée contre une paroi latérale interne (23) du boîtier (2).

3. Auto-injecteur (1) selon l'une des revendications précédentes 1 et 2, dans lequel la languette de verrouillage (6) a une nervure axiale (63) s'étendant de manière distale depuis l'extrémité proximale libre (60) de la languette de verrouillage (6), et ladite nervure axiale (63) est fixée à une paroi externe (51) du dispositif de retenue (3).

4. Auto-injecteur (1) selon la revendication précédente, dans lequel la nervure axiale (63) a une extrémité distale (630) qui est fixée à un élément de liaison (54) qui relie une paroi externe (51) de capuchon (5) à un dispositif de retrait (50) du capuchon (5).

5. Auto-injecteur (1) selon l'une des revendications précédentes 1 à 4, dans lequel la languette de verrouillage (6) a un renfort (64) agencé à un côté externe de l'extrémité proximale libre (60), le renfort (64) faisant saillie de manière proximale depuis un bord distal (510) du capuchon (5).

6. Auto-injecteur (1) selon la revendication précédente, dans lequel le renfort (64) comprend une surface inclinée (641) pour faciliter l'insertion de la languette de verrouillage (6) à l'intérieur du boîtier (2).

7. Auto-injecteur (1) selon l'une des revendications précédentes, dans lequel le dispositif de retenue (3) comprend une patte flexible (37), ladite patte flexible (37) ayant une extrémité fixe (35) fixée au boîtier (2) et une extrémité libre (36) définissant la surface de butée distale (30) du dispositif de retenue (3).

8. Auto-injecteur (1) selon la revendication précédente, dans lequel l'extrémité libre (36) de la patte flexible (37) est distale par rapport à l'extrémité fixe (35).

9. Auto-injecteur (1) selon l'une des revendications précédentes 7 et 8, dans lequel l'extrémité fixe (35) de la patte flexible (37) comprend une nervure annulaire (34) faisant saillie radialement depuis une paroi latérale interne (23) du boîtier (2).

10. Auto-injecteur (1) selon l'une des revendications précédentes 7 à 9, dans lequel la position de blocage du dispositif de retenue (3) est une position de repos de la patte flexible (37).

11. Auto-injecteur (1) selon l'une des revendications précédentes 7 à 9, dans lequel la position de libération du dispositif de retenue (3) est une position de repos.

12. Auto-injecteur (1) selon l'une des revendications précédentes, dans lequel le dispositif de retenue (3) a une surface de butée proximale (32) venant en butée contre une surface de butée distale (44) du couvercle d'aiguille (4) afin de bloquer le couvercle d'aiguille (4) dans la direction distale.

13. Auto-injecteur (1) selon l'une des revendications précédentes, dans lequel l'auto-injecteur (1) comprend des moyens de fixation pour fixer de manière amovible le capuchon (5) au boîtier (2) dans les directions axiale et de rotation.
